# EUROPÄISCHE PATENTSCHRIFT

(11) **EP 1 074 274 B1**
(45) Veröffentlichungstag und Bekanntmachung des Hinweises auf die Patenterteilung: **21.06.2006**
(21) Anmeldenummer: 00250263.1
(22) Anmeldetag: 04.08.2000
(51) Int. Cl.: A61N 1/37

(54) **Vorrichtung zur Erkennung von Fusionsereignissen bei Elektrostimulation des Herzens**
Device for detecting fusion events during cardiac electrostimulation
Dispositif pour la détection d'événements de fusion pendant l'électrostimulation cardiaque

(30) Priorität: 06.08.1999 DE 19938376
(43) Veröffentlichungstag der Anmeldung: 07.02.2001
(73) Patentinhaber: BIOTRONIK GmbH & Co. KG, 12359 Berlin (DE); Cortronik Mess- und Therapiegeräte GmbH, 8010 Graz (AT)
(72) Erfinder: Hutten, Helmut, 8010 Graz (AT)
(74) Vertreter: Lindner-Vogt, Karin L.

(56) Entgegenhaltungen:
- EP-A- 0 570 895
- WO-A-99/65569

## Beschreibung

Die Erfindung betrifft eine kardiologische Vorrichtung mit einem Sensor zur Aufnahme elektrischer Signale vom Herzen und mit Signalverarbeitungsmitteln, die mit dem Sensor verbunden und zur Verarbeitung vom Sensor empfangegener Signale ausgebildet sind und Vorverarbeitungsmittel umfassen. Aus dem Stand der Technik sind Vorrichtungen bekannt, die entweder zur Erkennung von Spontanerregungen des Herzens oder zur Erkennung von durch einen elektrischen Reiz ausgelösten stimulierten Erregungen des Herzens verwendet werden.

Diese bekannten Vorrichtungen gehen davon aus, daß im zeitlichen Verlauf jenes elektrischen Signales des Herzens, das den Vorgang der Erregung begleitet, typische Merkmale enthalten sind, die eine Unterscheidung zwischen Spontanerregungen und stimulierten Erregungen ermöglichen.

Elektrische Herzschrittmacher unterschiedlicher Ausführungsformen dienen der Stimulation des Herzens in Fällen, in denen entweder eine Störung der physiologischen Entstehung und/oder Ausbreitung der Erregung vorliegt oder therapeutische Überlegungen die Stimulation von Erregungen indizieren. Die zeitliche Ablaufsteuerung des elektrischen Herzschrittmachers muß in der Lage sein, zu erkennen, ob und wann eine spontane Erregung auftritt. Insbesondere darf es durch eine nicht zeitgerechte Stimulation zu keiner Beeinträchtigung der Pumpaktion des Herzens, die durch die Erregung bewirkt wird, kommen.

Im Herzen sind der Ort der spontanen Erregungsbildung und der Ort, wo die spontane Erregungsbildung meßtechnisch erfaßt wird, räumlich getrennt. Üblicherwerise werden ventrikuläre Schrittmacher - Elektroden in der Spitze des Ventrikels implantiert. Von einem Schrittmacher durch elektrische Stimulation in der Spitze des Ventrikels ausgelöste ventrikuläre Kontraktionen setzen sich in einer Erregungsfront fort, die von der Spitze des Ventrikel auf dem gesamten Umfang des Ventrikels in Richtung des AV-Knotens eines Herzens wandert. Vom Herzen selber ausgelöste ventrikuläre Kontraktionen des Ventrikels widerum stammen aus dem Sinusknoten im Atrium des Herzenz und werden über den AV-Knoten in das Ventrikel übergeleitet. Daraus ergibt sich, daß bei einer vom Herzen selber verursachten Kontraktion die ventrikuläre Erregungsfront vom AV-Knoten ausgelöst wird und in Richtung der Spitze des Ventrikels wandert. Demzufolge kann sie an der dort plazierten Schrittmacherelektrode auch erst relativ spät nach Beginn ihres Auftretens erfaßtwerden, nämlich nach Ablauf der sogenannten Latenzzeit. Da sich eine Erregung im Herzen mit relativ geringer Geschwindigkeit ausbreitet, kann es vorkommen, daß eine elektrische Stimulation erfolgt, obwohl bereits eine Spontanerregung aufgetreten ist, da die Spontananregegung zu demjenigen Zeitpunkt noch nicht erfolgt war, als die elektrische Stimulation ausgelöst wurde. In diesem Fall laufen zwei Erregungsfronten, die eine ausgehend vom Ort der Spontanerregung und die andere vom Ort der elektrischen Stimulation, aufeinander zu. Die Erregung eines Teiles des Herzgewebes wird durch die Spontanerregung, die Erregung des anderen Teiles des Herzgewebes durch die elektrische Stimulation bewirkt. Derartige Ereignisse werden als Fusionsereignisse bezeichnet. Fusionsereignisse können die der Pumpaktion des Herzens zugrundeliegende elektrophysiologische Koordination, damit den mechanischen Ablauf und somit die Pumpleistung beeinflussen.

Aus EP 0 570 895 ist Vorrichtung zum Erfassen abnomen Herzrythmen bekannt. In dieser Veroffentlichtung findet sich kein Hinweis auf Fusionereignisse and damit verbundener Probleme.

Der Erfindung liegt die Aufgabe zugrunde, derartige Fusionsereignisse zu erkennen.

Diese Aufgabe wird erfindungsmäß durch eine Vorrichtung der eingangs genannten Art gelöst, deren Signalverarbeitungsmittel die im zweiten Teil des Anspruchs 1 genannten Merkmale umfassen.

Vorteilhafte Ausführungsvarianten zeichen sich fakultativ durch die folgenden Merkmale aus:
- daß die Parameterbestimmungsmittel zum Ableiten des Parameters aus dem jeweils zuletzt vom Sensor erfaßten Signalabschnitt ausgebildet sind.
- daß die Parameterbestimmungsmittel zum Ableiten des Parameters aus dem gemittelten Signalabschnitt ausgebildet sind.

- daß die Signalverarbeitungsmittel Vergleichsmittel umfassen, die mit den Parameterbestimmungsmitteln und einem Grenzwertspeicher verbunden zum Vergleich des abgeleiteten Parameters und einem im Grenzwertspeicher gespeicherten Grenzwert sowie zur Abgabe eines Fusionssignals ausgebildet sind.
- daß die Signalverarbeitungsmittel Streuwertermittlungsmittel umfassen, die zum Ausgeben eines Streuwertes für zeitlich aufeinanderfolgende Signale ausgebildet und mit den Vorverarbeitungsmitteln verbunden sind.
- daß die Streuwertermittlungsmittel mit dem Mittelwertspeicher verbunden sind.
- daß die Signalverarbeitungsmittel zusätzlich Grenzwertbestimmungsmittel umfassen, die zum Bestimmen des Grenzwertes aus den Streuwerten ausgebildet mit den Streuwertermittlungsmitteln sowie einem Grenzwertspeicher verbunden sind. Der Grenzwertspeicher kann dabei integraler Bestandteil der Grenzwertbestimmungsmittel sein.
- daß Vorverarbeitungsmittel einen Momentansignalspeicher für den jeweils zuletzt vom Sensor erfaßten Signalabschnitt umfassen. In diesem Fall greifen die der Signalvorverarbeitung nachgeschalteten Einheiten zur Parameterermittlung und zur Mittelwertbeildung sowie zur Streuwertermittlung auf den Momentansignalspeicher der Vorverarbeitungsmittel zu.
- daß die Signalverarbeitungsmittel Inhibitormittel umfassen, die zum Unterdrücken solcher Signalabschnitte für die weitere Signalverarbeitung ausgebildet sind, für die Vergleichsmittel ein Fusionssignal abgeben. Signale infolge von Fusionsereignissen werden somit für die Mittelwertbildung und fü die Streuwertermittlung nicht berücksichtigt.
- daß die Inhibitormiteel derart gestaltet sind, daß sie den Momentansignalspeicher löschen, wenn eine Fusionssignal auftritt.

Die Erfindung schließt die technische Lehre ein, daß bei der Erkennung eines Fusionsereignisses auch solche Zusammenhänge beachtet werden müssen, welche über eine unmittelbare Zuordnung zur mechanischen Leistung des Herzens hinausgehen. Insbesondere berücksichtigt die Erfindung die gesicherte Erkenntnis, daß sich individuelle Besonderheiten wie Form und Größe des Herzens, der Meßort für eine eventuell auftretende Spontandepolarisation und pathophysiologische Störungen, auf den Verlust des Meßsignales auswirken können.

Ferner liegt der Erfindung die Erkenntnis zugrunde, daß in einem biologischen System nacheinander auftretende Signale niemals die genau gleiche Signalfeinstruktur aufweisen, sondern gewisse Abweichungen aufweisen können, deren Ursache nicht ausschließlich das Auftreten eines Fusionsereignisses sein muß.

Bei den zur Erkennung von Fusionsereignissen herangezogenen Signalen handelt es sich um elektrische Signale, die in bekannter Weise mit einem Sensor gewonnen werden, welcher mit einer zur Vor- und Weiterverarbeitung dieser Signale geeigneten Vorrichtung in leitender Verbindung steht. Die Vorverarbeitung, z.B. die Verstärkung und die frequenzbestimmende Filterung zur Befreiung von Signalanteilen, die im Sinne der Erkennung eines Fusionsereignisses nicht informationsrelevant sind, kann in einer dem Stand der Technik entsprechenden Weise erfolgen. Einige der dem Stand der Technik entsprechenden implantierbaren Herzschrittmacher mit Sensingkanal sind zu dieser Vorverarbeitung befähigt. Die Vorverarbeitung des Signales kann in analoger oder in digitaler Form ausgeführt werden.

Der Erfindung liegt eine Vorrichtung zugrunde, die es gestattet, aus einem durch eine Herzerregung hervorgerufenen und geeignet weiterverarbeiteten elektrischen Signal solche Merkmalsparameter zu extrahieren, die für das Auftreten eines Fusionsereignisses kennzeichnend sind. Die Merkmalsextraktion wird vorteilhafterweise in digitaler Verarbeitung durchgeführt, ist jedoch grundsätzlich auch in analoger oder gemischter analog-digitaler Verarbeitungsweise möglich.

Die Erfindung wird durch eine Vorrichtung verwirklicht, die mit der Fähigkeit zur Mittelwertsbildung aus den in zeitlicher Folge nacheinander auftretenden Einzelsignalen ausgestattet ist. Die Mittelwertsbildung kann für den gesamten Verlauf der Einzelsignale, soweit dieser zur Erkennung von Fusionsereignissen relevant ist, oder für bestimmte Merkmale, die aus den Einzelsignalen gewonnen wurden und für die Erkennung von Fusionsereignissen relevant sind, durchgeführt werden. Bei den aus den Einzelsignalen gewonnenen Merkmalen kann es sich um ein oder mehrere Merkmale handeln.

Der Ermittlung des Mittelwertes kann eine zeitliche Bewertungsfunktion zugrundegelegt werden. Ferner besitzt die Vorrichtung die Fähigkeit, aus der Abweichung der Einzelsignale oder der daraus erhaltenen Merkmale vom Mittelwert einen Streuwert zu bilden. Dieser Streuwert ist ein Maß für die Abweichung der Einzelsignale und der daraus erhaltenen Merkmale vom Mittelwert. Durch diese Mittelwertsbildung wird das Ziel einer ausreichenden Berücksichtigung individueller Besonderheiten erreicht.

Als Fusionsereignisse klassifizierte Einzelsignale werden weder bei der Bildung des Mittelwertes noch bei der Berechnung des Streuwertes berücksichtigt. Da die Ermittlung des Mittelwertes und des Streuwertes fortlaufend vorgenommen wird, wird die im Sinne des Erfindungsanspruchs wesentliche Anforderung der zeitlichen Adaption an sich verändernde Verhältnisse, die Auswirkungen auf die Signalfeinstruktur haben, erfüllt.

Jedes neu auftretende Einzelsignal wird mit dem gespeicherten Mittelwert hinsichtlich jener Merkmale verglichen, die für ein Fusionsereignis kennzeichnend sind. Wenn die Abweichung einen Wert überschreitet, der basierend auf dem Streuwert als Grenzwert festgelegt ist, wird das Ereignis als Fusionsereignis klassifiziert.

Gegenstand der Erfindung ist somit eine Vorrichtung zur Erkennung von Fusionsereignissen, die durch die Stimulation des Herzens mit einem elektrischen Herzschrittmacher hervorgerufen werden, wobei die Vorrichtung Merkmalsparameter, die für Fusionsereignisse kennzeichnend sind, auf der Basis einer fortlaufenden Bildung von Mittelwerten mit beliebig einstellbarer zeitlicher Bewertungsfunktion ermittelt, mit den aus dem zuletzt aufgetretenen Einzelsignal abgeleiteten entsprechenden Merkmalsparametern vergleicht und die Abweichung von einem Grenzwert auf der Basis eines Streuwertes, der ebenfalls von der erfindungsgemäßen Vorrichtung fortlaufend ermittelt wird, als Entscheidungskriterium für ein Fusionsereignis verwendet.

Diese Vorrichtung zeichnet sich fakultativ durch die Merkmale aus:
- daß als Merkmalsparameter zur Erkennung eines Fusionsereignisses der Zeitpunkt des ersten Nulldurchganges des mit dem Sensor (1) festgestellten elektrophysiologischen Signales der Herzerregung verwendet wird;
- daß als Merkmalsparameter zur Erkennung eines Fusionsereignisses die Steilheit des mit dem Sensor (1) festgestellten elektrophysiologischen Signales der Herzerregung im Zeitpunkt des ersten Nulldurchganges verwendet wird;
- daß als Merkmalsparameter zur Erkennung eines Fusionsereignisses der Zeitpunkt jenes Knickes im Verlauf des mit dem Sensor (1) festgestellten elektrophysiologischen Signales der Herzerregung verwendet wird, zu dem der zunächst schnelle Verlauf der Depolarisation deutlich in einen langsameren Verlauf übergeht;
- daß als Merkmalsparameter zur Erkennung eines Fusionsereignisses jener Zeitpunkt verwendet wird, zu dem das mit dem Sensor festgestellte elektrophysiologische Signal der Herzerregung ein Maximum aufweist;
- daß als Merkmalsparameter zur Erkennung eines Fusionsereignisses jener Zeitpunkt verwendet wird, zu dem das mit dem Sensor festgestellte elektrophysiologische Signal der Herzerregung nach abgelaufener Erregung wieder zu Null wird;
- daß als Merkmalsparameter zur Erkennung eines Fusionsereignisses die maximale Steilheit im Verlauf des mit dem Sensor festgestellten elektrophysiologischen Signales der Herzerregung beim Rückgang zum Ausgangssignal nach abgelaufener Herzerregung oder ein aus der maximalen Steilheit abgeleiteter Hilfsparameter verwendet wird;
- daß mehrere Merkmalsparameter ermittelt und in zusammenfassender Bewertung als Kriterium für das Auftreten eines Fusionsereignisses verwendet werden;
- daß der Sensor zur Erfassung des elektrophysiologischen Signales der Herzerregung zugleich als Elektrode zur Stimulation des Herzens verwendet wird;
- daß der Sensor zur Erfassung des elektrophysiologischen Signales der Herzerregung aus mehreren Komponenten besteht;
- daß die Vorrichtung in einem dafür geeigneten Gehäuse vollständig im Körper eines Menschen implantiert wird;
- daß die Vorrichtung gemeinsam mit einem elektrischen Herzschrittmacher in einem Gehäuse untergebracht und vollständig im Körper eines Menschen implantiert wird.

Die Erfindung soll nun anahnd eines Ausführungsbeispiels mit Hilfe der Figur näher erläutert werden.

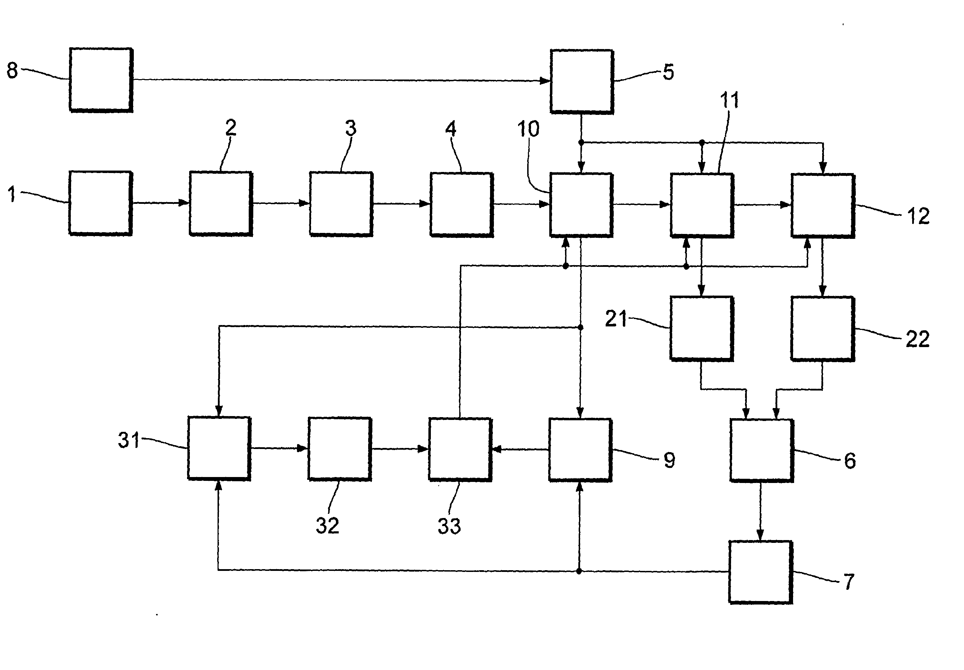

Figur 1 zeigt die wesentlichen Funktionseinheiten und ihre funktionsgerechte Anordnung, mit der der Oberbegriff des Anspruches verwirklicht werden kann. Die im Text in Klammern zu den Funktionseinheiten aufgeführten Ziffern entsprechen den in Figur 1 zur Bezeichnung der Funktionseinheiten verwendeten Ziffern.

Das mit dem Sensor (1) aufgenommene Signal als Ausdruck der Herzerregung wird nach geeigneter Vorverarbeitung, die im vorliegenden Beispiel in analoger Form mit einem Eingangsverstärker (2) und einem Filter (3) erfolgt, einem Analog-DigitalWandler (4) zugeführt. Die Analog-Digital-Wandlung muß mit einer ausreichend hohen Abtastrate und mit ausreichend hoher Amplitudendiskretisierung vorgenommen werden, so daß dadurch keine Verfälschung bzw. Elimination der für eine Fusionserkennung wesentlichen Signalanteile bewirkt wird.

Das auf diese Weise erhaltene digitalisierte Signal wird einem ersten Speicher (10) zugeführt, der aus einer hinreichenden Zahl von Speicherzellen bestehen muß, damit er alle für die weitere Verarbeitung erforderlichen Signalwerte aufnehmen kann. Die Abspeicherung der zeitlich nacheinander auftretenden Signalwerte erfolgt in geordneter Form. Nach dem ersten Speicher (10) ist ein zweiter, dem ersten weitgehend entsprechend aufgebauter Speicher (11) angeordnet. Der Inhalt des ersten Speichers (10) kann durch einen Steuerimpuls, der in der Speichersteuerung (5) erzeugt wird, in geordneter Form in den zweiten Speicher (11) übernommen werden. Nach dem zweiten Speicher (11) kann ein dritter, entsprechend wie Speicher (11) aufgebauter Speicher (12) angeordnet sein, der mit dem Auftreten des Steuerimpulses am Ausgang der Speichersteuerung (5) den Inhalt des zweiten Speichers (11) in geordneter Form übernimmt.

Die Zahl N der in dieser Weise hintereinander angeordneten Speicher kann beliebig groß sein, muß jedoch mindestens gleich 2 sein. Mit dem Auftreten des Steuerimpulses am Ausgang der Speichersteuerung (5) übernimmt jeder Speicher in geordneter Weise den Inhalt des vorgeschalteten Speichers, lediglich der Inhalt des an letzter Stelle stehenden Speichers geht mit dem Auftreten des Steuerimpulses verloren.

Die in den hintereinander angeordneten Speichern (11, 12 ... 1N) mit Ausnahme des ersten Speichers (10) enthaltenen Signalanteile werden über Bewertungsvorrichtungen (21, 22 ... 2N) einer Addiervorrichtung (6) zugeführt. Dabei ist jede der Bewertungsvorrichtungen in gleicher Weise mit einem der Speicher verbunden, z.B. der zweite Speicher (11) mit der Bewertungsvorrichtung (21), der dritte Speicher (12) mit der Bewertungsvorrichtung (22) usw.. Insgesamt sind also ebensoviele Bewertungsvorrichtungen wie Speicher mit Ausnahme des ersten Speichers (10) vorhanden. Allerdings kann jede Bewertungsvorrichtung mit einem eigenen Bewertungsfaktor zwischen 1 und 0 mit geeigneter Unterteilung ausgestattet werden. Damit kann der Inhalt jedes Speichers (11, 12 ... 1 N) mit einem einstellbaren Bewertungsfaktor multiplikativ gewichtet werden, bevor er der Addiervorrichtung (6) zugeführt wird. Der Addiervorrichtung nachgeschaltet ist ein weiterer Speicher (7), der wie die Speicher (11, 12 ... 1N) aufgebaut ist. Der Inhalt dieses Speichers (7) enthält also nach durchgeführter Addition ein Signal, das den mit dem Auftreten jedes Einzelsignales neu gebildeten und auf der Basis einer beliebig einstellbaren Gewichtungsfunktion ermittelten Mittelwert der in den Speichern (11, 12 ... 1N) enthaltenen Signale darstellt. Der zeitliche Bezugswert, der für die zeitliche Zuordnung der verschiedenen Signalwerte der nacheinander auftretenden Einzelsignale und damit für eine korrekte Mittelwertbildung erforderlich ist, wird aus der Vorderflanke jenes Stimulus abgeleitet, der vom Ausgangsverstärker (8) des elektrischen Herzschrittmachers abgegeben wird.

Der Vergleich zwischen dem Mittelwertssignal in Speicher (7) und dem neu aufgetretenen Signal in Speicher (10) kann sich auf jedes Merkmal beziehen, das für ein Fusionsereignis kennzeichnend ist. Dabei kann es sich ebenso um zeitliche Merkmale, z.B. das Auftreten charakteristischer Punkte im Signalverlauf wie Nulldurchgänge, Maxima, Minima, Zeitpunkte größter positiver oder negativer Steigung im Signalverlauf oder um Unterschiede in der Signalmorphologie handeln. Die Vorrichtung zur quantitativen Ermittlung dieses Merkmalparameters (9) verwendet Schaltungen entsprechend dem Stand der Technik, z.B. zur Bestimmung eines Nulldurchgangs einen Amplitudendiskriminator oder zur Ermittlung des Zeitpunktes von Maxima, Minima oder Steigungsparametern den nach der Zeit differenzierten Signalverlauf. Unterschiede in der Signalmorphologie können mit Korrelationsschaltungen ermittelt werden. Vorteilhafterweise kann die Vorrichtung zur quantitativen Ermittlung dieses Merkmalsparameters (9) durch eine Mikroprozessorschaltung verwirklicht werden.

Da in einem biologischen System nacheinander auftretende Einzelsignale niemals identisch sind, wird zusätzlich aus den in den Speichern (11, 12 ...1 N) gespeicherten Signalen in der Streuwert-Ermittlungsvorrichtung (31) ein Streuwert gebildet für jenes Merkmal, das für die Fusionserkennung verwendet wird. Dieser Streuwert stellt ein Maß dar für die Streubreite der Einzelsignale ohne Fusionsereignisse um jenen Merkmalswert, der adaptiv gebildet und wegen der Nichtberücksichtigung von Fusionsereignissen als Bezugswert für Nicht-Fusionsereignisse anzusehen ist. Die Streuwert-Ermittlungsvorrichtung (31) kann durch Schaltungen verwirklicht werden, die dem Stand der Technik entsprechen, vorteilhafterweise durch eine Mikroprozessorschaltung.

Der in (31) ermittelte Streuwert wird in einer Bewertungsschaltung (32) in jenen Grenzwert umgewandelt, dessen Überschreitung kennzeichnend für ein Fusionsereignis ist. In der Komparatorschaltung (33) wird der in der Vorrichtung zur quantitativen Ermittlung des Merkmalsparameters (9) ermittelte Wert mit jenem verglichen, der als Grenzwert in der Bewertungsschaltung (32) ermittelt worden ist. Ein Fusionsereignis liegt dann vor, wenn das Ausgangssignal von (9) größer ist als jenes von (32). Wenn ein Fusionsereignis festgestellt worden ist, wird der Inhalt des Speichers (10) auf Null gestellt und eine Weitergabe des Speicherinhaltes der Speicher (11, 12 ... 1N) verhindert. Damit werden der Inhalt des Mittelwertspeichers (7) sowie der Streuwert-Ermittlungsvorrichtung (31) durch ein Fusionsereignis nicht verändert.

## Patentansprüche

1. Kardiologische Vorrichtung mit einem Sensor (1) zur Aufnahme elektrischer Signale vom Herzen und mit Signalverarbeitungsmitteln, die mit dem Sensor (1) verbunden und zur Verarbeitung vom Sensor (1) empfangegener Signale ausgebildet sind und Vorverarbeitungsmittel (2, 3, 4) sowie einen Bezugszeitpunktgeber (8) umfassen, der zum Abgeben eines Zeitsignals bei Auftreten eines Periodisch wiederkehrenden Signalmerkmals ausgebildet ist, **dadurch gekennzeichnet, dass** die Signalverarbeitungsmittel
- Mittelwertbildungsmittel (11, 12, 21, 22, 6) umfassen, die zur Bildung eines über mehrere, zwischen jeweils zwei Zeitsignalen liegenden Signalabschnitte gemittelten Signalabschnitts ausgebildet und mit den Vorverarbeitungsmitteln (2, 3, 4) sowie dem Bezugszeitpunktgeber (8) verbunden sind, sowie
- einen Mittelwertspeicher (7), der zur Speicherung der Mittelwerte ausgebildet und mit den Mittelwertbildungsmitteln verbunden ist,und
- Parameterbestimmungsmittel (9), die mit den Vorverarbeitungsmitteln (2, 3, 4) und mit dem Mittelwertspeicher verbunden und zur Ermittlung eines einen Signalabschnitt kennzeichnenden Parameters ausgebildet sind, und
- Streuwertermittlungsmittel (31), die zum Ausgeben eines Streuwertes für zeitlich aufeinanderfolgende Signale ausgebildet und mit den Vorverarbeitungsmitteln (2, 3, 4) verbunden sind, und
- eine Komparatorschaltung (33), die wenigstens mittelbar mit dem Parameterbestimmungsmittel (9) und dem Streuwertermittlungsmittel (31) verterbestimmungsmittel (9) und dem Streuwertermittlungsmittel (31) verbunden und ausgebildet ist, den Parameter mit einem aus dem Streuwert abgeleiteten Grenzwert zu vergleichen und bei Überschreiten des Grenzwertes ein ein Fusionsereignis kennzeichnendes Signal zu erzeugen.

2. Vorrichtung nach Anspruch 1, **dadurch gekennzeichnet, daß** die Parameterbestimmungsmittel (9) zum Ableiten des Parameters aus dem jeweils zuletzt vom Sensor (1) erfaßten Signalabschnitt ausgebildet sind.

3. Vorrichtung nach Anspruch 1 oder 2, **dadurch gekennzeichnet, daß** die Parameterbestimmungsmittel (9) zum Ableiten des Parameters aus dem gemittelten Signalabschnitt ausgebildet sind.

4. Vorrichtung nach einem der Ansprüche 1 bis 3, **dadurch gekennzeichnet, daß** die Signalverarbeitungsmittel Vergleichsmittel (33) umfassen, die mit den Parameterbestimmungsmitteln (9) und einem Grenzwertspeicher verbunden zum Vergleich des abgeleiteten Parameters und einem im Grenzwertspeicher gespeicherten Grenzwert sowie zur Abgabe eines Fusionssignals ausgebildet sind.

5. Vorrichtung nach einem der Ansprüche 1 bis 4, **dadurch gekennzeichnet, daß** die Streuwertermittlungsmittel (31) mit dem Mittelwertspeicher (7) verbunden sind.

6. Vorrichtung nach einem der Ansprüche 1 bis 5, **dadurch gekennzeichnet, daß** die Signalverarbeitungsmittel zusätzlich Grenzwertbestimmungsmittel (32) umfassen, die zum Bestimmen des Grenzwertes aus den Streuwerten ausgebildet mit den Streuwertermittlungsmitteln (31) sowie einem Grenzwertspeicher verbunden sind.

7. Vorrichtung nach einem der Ansprüche 1 bis 6, **dadurch gekennzeichnet, daß** Vorverarbeitungsmittel (2, 3, 4) einen Momentansignalspeicher (10) für den jeweils zuletzt vom Sensor (1) erfaßten Signalabschnitt umfassen.

8. Vorrichtung nach Anspruch 4 und einem der Ansprüche 1 bis 7, **dadurch gekennzeichnet, daß** die Signalverarbeitungsmittel Inhibitormittel umfassen, die zum Unterdrücken solcher Signalabschnitte für die weitere Signalverarbeitung ausgebildet sind, für die Vergleichsmittel (33) ein Fusionssignal abgeben.

9. Vorrichtung nach Anspruch 7 und 8, **dadurch gekennzeichnet, daß** die Inhibitormittel derart gestaltet sind, daß sie den Momentansignalspeicher (10) löschen, wenn eine Fusionssignal auftritt.

## Claims

1. A cardiological device having a sensor (1) for recording electrical signals from the heart and having signal processing means, which are connected to the sensor (1) and are implemented to process signals received by the sensor (1) and comprise preprocessing means (2, 3, 4) and a reference instant encoder (8), which is implemented to output a time signal upon the occurrence of a periodically returning signal feature, **characterized in that** the signal processing means comprise
- average value calculation means (11, 12, 21, 22, 6), which are implemented to calculate a signal section average over multiple signal sections lying between two time signals and are connected to the preprocessing means (2, 3, 4) and the reference instant encoder (8), and
- an average value memory (7), which is implemented to store the average values and is connected to the average value calculation means, and
- parameter determination means (9), which are connected to the preprocessing means (2, 3, 4) and to the average value memory and are implemented to ascertain a parameter characterizing a signal section, and
- scattered value ascertainment means (31), which are implemented to output a scattered value for chronologically sequential signals and are connected to the preprocessing means (2, 3, 4), and
- a comparator circuit (33), which is at least indirectly connected to the parameter determination means (9) and the scattered value ascertainment means (31) and is implemented to compare the parameter to the limiting value derived from the scattered value and, if the limiting value is exceeded, to generate a signal identifying a fusion event.

2. The device according to Claim 1, **characterized in that** the parameter determination means (9) are implemented to derive the parameter from the particular last signal section detected by the sensor (1).

3. The device according to Claim 1 or 2, **characterized in that** the parameter determination means (9) are implemented to derive the parameter from the averaged signal section.

4. The device according to one of Claims 1 through 3, **characterized in that** the signal processing means comprise comparison means (33), which are connected to the parameter determination means (9) and the limiting value memory, to compare the derived parameter and a limiting value stored in the limiting value memory and to output a fusion signal.

5. The device according to one of Claims 1 through 4, **characterized in that** the scattered value ascertainment means (31) are connected to the average value memory (7).

6. The device according to one of Claims 1 through 5, **characterized in that** the signal processing means additionally comprise limiting value determination means (32), which are implemented to determine the limiting value from the scattered values and are connected to the scattered value ascertainment means (31) and a limiting value memory.

7. The device according to one of Claims 1 through 6, **characterized in that** preprocessing means (2, 3, 4) comprise an instantaneous signal memory (10) for the particular last signal section detected by the sensor (1).

8. The device according to Claim 4 and one of Claims 1 through 7, **characterized in that** the signal processing means comprise inhibitor means, which are implemented to suppress those signal sections for further signal processing which output a fusion signal for the comparison means (33).

9. The device according to Claims 7 and 8, **characterized in that** the inhibitor means are designed in such a way that they erase the instantaneous signal memory (10) when a fusion signal occurs.

## Revendications

1. Dispositif cardiologique comportant un capteur (1) servant à recevoir des signaux électriques du coeur et des moyens de traitement de signaux qui sont reliés au capteur (1) et conçus pour traiter les signaux reçus du capteur (1) et englobent des moyens de traitement préalable (2, 3, 4) ainsi qu'un indicateur de moment de référence (8) qui est conçu pour émettre un signal temporel lors de la survenance d'une caractéristique de signal revenant périodiquement, **caractérisé en ce que** les moyens de traitement de signaux comprennent
- des moyens de calcul de valeurs moyennes (11, 12, 21, 22, 6) qui sont conçus pour calculer une moyenne de sections de signaux sur plusieurs sections de signaux se trouvant respectivement entre deux signaux temporels et sont reliés aux moyens de traitement préalable (2, 3, 4) ainsi qu'à l'indicateur de moment de référence (8),
- ainsi qu'une mémoire de valeurs moyennes (7) qui est conçue pour sauvegarder les valeurs moyennes et est reliée aux moyens de calcul de valeurs moyennes,
- et des moyens de détermination de paramètres (9) qui sont reliés aux moyens de traitement préalable (2, 3, 4) et à la mémoire de valeurs moyennes et sont conçus pour déterminer un paramètre caractérisant une section de signaux et que
- des moyens de détermination de valeurs de dispersion (31) qui sont conçus pour émettre une valeur de dispersion pour les signaux successifs dans le temps et sont reliés aux moyens de traitement préalable (2, 3, 4),
et un circuit comparateur (33) qui est relié du moins indirectement au moyen de détermination de paramètres (9) et au moyen de détermination de valeurs de dispersion (31) et est conçu pour comparer le paramètre à une valeur limite dérivée d'une valeur de dispersion et, en cas de dépassement de la valeur limite, générer un signal caractérisant un phénomène de fusion.

2. Dispositif selon la revendication 1, **caractérisé en ce que** les moyens de détermination de paramètres (9) sont conçus pour dériver le paramètre de la section de signaux enregistrée respectivement en dernier par le capteur (1).

3. Dispositif selon la revendication 1 ou 2, **caractérisé en ce que** les moyens de détermination de paramètres (9) sont conçus pour dériver le paramètre de la moyenne de la section de signaux.

4. Dispositif selon une des revendications 1 à 3, **caractérisé en ce que** les moyens de traitement de signaux englobent des moyens de comparaison (33) qui, reliés aux moyens de détermination de paramètres (9) et à une mémoire de valeurs limites, sont conçus pour comparer le paramètre dérivé et une valeur limite sauvegardée dans la mémoire de valeurs limites ainsi que pour émettre un signal de fusion.

5. Dispositif selon une des revendications 1 à 4, **caractérisé en ce que** les moyens de détermination de valeurs de dispersion (31) sont reliés à la mémoire de valeurs moyennes (7).

6. Dispositif selon une des revendications 1 à 5, **caractérisé en ce que** les moyens de traitement de signaux comprennent en outre des moyens de détermination de valeurs limites (32) qui, conçus pour déterminer la valeur limite à partir des valeurs de dispersion, sont reliés aux moyens de détermination de valeurs de dispersion (31) ainsi qu'à une mémoire de valeurs limites.

7. Dispositif selon une des revendications 1 à 6, **caractérisé en ce que** les moyens de traitement préalable (2, 3, 4) comprennent une mémoire de signaux momentanés (10) pour la section de signaux respectivement enregistrée en dernier par le capteur (1).

8. Dispositif selon la revendication 4 et une des revendications 1 à 7, **caractérisé en ce que** les moyens de traitement de signaux comprennent des moyens inhibiteurs qui sont conçus pour inhiber de telles sections de signaux pour le traitement ultérieur des signaux pour lesquels les moyens de comparaison (33) émettent un signal de fusion.

9. Dispositif selon les revendications 7 et 8, **caractérisé en ce que** les moyens inhibiteurs sont réalisés de manière à effacer la mémoire de signaux momentanés (10) lorsqu'un signal de fusion survient.
